# EUROPEAN PATENT APPLICATION

(11) **EP 0 804 921 A1**
(43) Date of publication of application: **05.11.1997**
(21) Application number: 97302015.9
(22) Date of filing: 25.03.1997
(51) Int. Cl.: A61K 7/32

(54) **Topical composition**

(30) Priority: 02.04.1996 GB 9606901
(71) Applicant: UNILEVER PLC, London EC4P 4BQ (GB); UNILEVER N.V., 3013 AL Rotterdam (NL)
(72) Inventor: Correia, Martinho, 60270 Govieux (FR); Hall, Lynne, Sherburn-in-Elmet, Leeds, L25 6LF (GB); Hagan, Desmond Bernard, South Wirral, Cheshire, L66 5NR (GB)
(74) Representative: Rots, Maria Johanna Francisca

(57) **Abstract**

An antiperspirant or deodorant composition for topical application to the human skin, comprising an antiperspirant or deodorant active material and a cosmetically suitable vehicle, the cosmetically suitable vehicle comprising 20-90% by weight of the total composition of a poly olefin emollient.

## Description

This invention relates to cosmetic compositions suitable for topical application to the human skin. In particular, it relates to antiperspirant and deodorant compositions for topical application to any appropriate area of the human body, such as the axilla.

Antiperspirant compositions have long been known for topical application to the body, in particular the axilla, for the control of perspiration and/or odour. The form of such products is many and varied; they may typically take the form of for example a propellant driven aerosol composition, a lotion, a stick, a cream, or a pumpspray formulation. Antiperspirant compositions will often contain an antiperspirant active salt, which is typically an aluminium or aluminium/zirconium salt, which acts to prevent the pores of the skin secreting sweat, in a topically acceptable vehicle.

Deodorant compositions for topical application to the human body are likewise found in many forms. They may have any of the product forms described above, and will typically comprise a deodorant agent in a cosmetically acceptable vehicle. The deodorant agent may for example be an inorganic salt which has a deodorant effect, such as a zinc or aluminium salt, or may be organic. Examples of organic deodorant agents include short chain monohydric alcohols such as ethanol, polyhydric alcohols such as propylene glycol, or compounds such as triclosan.

Over recent years, topically acceptable vehicles for such products have typically been based on water, short chain monohydric alcohols such as ethanol, or volatile silicone materials. Such vehicles have typically proved sensorially acceptable not least of all because of their relative volatility and low viscosity; once the product has been delivered to the skin, the cosmetic vehicle evaporates relatively quickly, leaving the user with a dry, pleasurable, comfortable sensation after product application.

Attempts have been made to use other cosmetic vehicles for use with such products. For example, attempts have been made to use mineral oils as cosmetic vehicles, for example for antiperspirant compositions. However, because of the non volatile nature of mineral oils, such products in which the major part of the cosmetic vehicle is a mineral oil have proved sensorially unacceptable, not least of all because of the greasy or sticky feeling they can leave on the users skin.

It is also known to use minor amounts (for example less than about 20%) of certain non volatile hydrocarbon polymer materials in cosmetic compositions, for example antiperspirant compositions, for the beneficial properties such materials can bring to the composition. For example, in their trade literature supporting the composition ETHYLFLO (now SILKFLO), Ethyl Corporation (now Albermarle Corporation) have advocated the use of polydecene materials in stick antiperspirants. Polydecenes are based on 1-decene, and various commercially available polydecene compositions can be obtaining having varying degrees of polymerization between the 1-decene units, with for example the commercially available polydecene materials containing various amounts of dimers, trimers, etc. In advocated formulations, 6% of polydecene is alleged to be acceptable to be introduced into an antiperspirant stick formulation, with the polydecene providing the stick with both emollient and skin moisturizing benefits.

However, such poly olefin materials (of which polydecene is an example) have not been used in antiperspirant or deodorant compositions previously where they make up the major part of the cosmetic vehicle of the composition. This is thought to be due to a general prejudice in the art against using excessive amounts of non volatile hydrocarbon materials such as poly olefins in antiperspirant or deodorant compositions; as explained above, such use would lead the skilled man to expect the product to have undesirable sensory properties, and be excessively greasy after application.

We have surprisingly found that poly olefin materials can be included in antiperspirant and deodorant compositions for topical application as the major part of the cosmetic vehicle, and yet result in products which have remarkably good sensory properties. Such products may prove to be remarkably lacking in greasiness on application.

In addition, where the composition is in the form of an antiperspirant composition, the inclusion of such poly olefin materials may act to a certain extent to mask any white deposits which may be caused by the presence of antiperspirant active salts in the composition, thereby improving the appearance of the applied product in use. It has also been found that the presence of such poly olefins does not interfere to a significant degree with the efficacy of the antiperspirant composition when applied.

Thus, according to the invention, there is provided an antiperspirant or deodorant composition for topical application to the human skin, comprising an antiperspirant or deodorant active material and a cosmetically suitable vehicle, the cosmetically suitable vehicle comprising 20-90% by weight of the total composition of a poly olefin emollient.

It is a particularly preferred form of the invention that the composition is an antiperspirant composition, and comprises an antiperspirant active material accordingly.

The poly olefins which make up the major part of the cosmetic vehicle according to the invention are hydrocarbon polymers, with the preferred ones being liquid at room temperature (i.e. 21°C). Many of the preferred poly olefins which comprises the major part of the cosmetic vehicle in compositions according to the invention will be non volatile. By "non volatile" in this context is meant that the emollient has a flash point of 140°C or greater. It is also highly preferred that the emollient in the composition has a relatively low viscosity. Preferably, the viscosity of the poly olefin hydrocarbon emollient is less than about 40 cSt at 40°C, more preferably less than about 30 cSt at 40°C. The poly olefin hydrocarbon emollient can be present in the composition at a level of 7-90% by weight, by is prefearbly present at a level of 20-90%, more preferably 30-90%, even more preferably 30-60% by weight of the composition.

Preferably, the poly olefin vehicle of compositions according to the invention comprises a poly alpha olefin. Preferred poly alpha olefin vehicles for use in compositions according to the invention are polydecenes, for example the Silkflo range of polydecenes, manufactured by Albermarle Corporation. Other preferred poly olefins for use in compositions according to the invention include polybutene, which is commercially available under the trade name Panalene L14E from Amoco, and polyisobutene, which can be obtained from Presperse under the trade name Permethyl.

As such, preferred poly olefins for use in compositions according to the invention may have chain lengths in the region of 3-15 carbon atoms, and in their unpolymerised form may have one or two double bonds. Preferred poly olefin blends which are commercially available may conveniently contain a blend of various polymers, including dimers, trimers, and so on. Preferred materials for use in compositions according to the invention include Silkflo 362NF, Silkflo 364NF, and Silkflo 366NF, available from Albermarle Corporation.

When used in compositions according to the invention, the poly olefin hydrocarbons described can act as the major part of the cosmetic vehicle for topical antiperspirant or deodorant compositions, yet confer to the composition surprisingly good sensory properties, including a surprisingly good lack of greasiness after application, and where the composition is an antiperspirant composition, even provide a degree of masking of any whiteness that may be left by the antiperspirant salt in the composition.

A further advantage of using the poly olefin hydrocarbons specified as the main part of the cosmetic vehicle is that they have been found not to interfere with the efficacy of any antiperspirant active salt in the composition to any major degree. Such interference with efficacy is a known problem in the art, and one which product formulators are constantly trying to overcome. The problem may for example be observed when certain other non volatile hydrocarbons form the major part of the cosmetic vehicle of such products, such as mineral oil, or isopropyl myristate. The deodorant agent used in compositions according to the invention may for example be an inorganic salt which has a deodorant effect, such as a zinc or aluminium salt, or may be organic. Examples of organic deodorant agents include short chain monohydric alcohols such as ethanol, polyhydric alcohols such as propylene glycol, or compounds such as triclosan.

Likewise, the antiperspirant agent used in compositions according to the invention may be any antiperspirant active which is used in such topical compositions. It may for example be a salt of aluminium, or aluminum/zirconium. Further suitable antiperspirant active materials are described in EP 28,853 (Procter & Gamble), the content of which is incorporated herein by reference.
Compositions according to the invention may take any convenient product form, including lotion, stick, cream, pump spray, or propellant driven aerosol.

A convenient form is a solid stick, which may conveniently be contained in a suitable holder or dispenser to enable it to be applied.

An alternative form is a lotion, which may conveniently be used in a roll ball dispenser, fitted with a ball valve, which may be applied to the skin in a conventional manner. A further possible composition would be a liquid suitable for dispensing from a finger operated pump spray or hand operated pump spray, which can deliver without the use of propellant gases a fine spray to the skin.

The composition can also take the form of a cream which is suitable or adapted for topical application to the skin.

A further possible product form is in the form of a liquid, which is suitable for application from a propellant driven aerosol by use of a suitable propellant, examples of which are well known in the art. It should be noted that, when the composition is in the form of a liquid to be applied from a propellant driven aerosol, the percentages of the components of the composition referred to, in particular the amount of poly olefin vehicle in the composition, refer to the so-called "concentrate" composition, before its dilution with propellant and dosage into the aerosol container.

Propellant driven aerosol compositions according to the invention will be packaged with an aerosol propellant. The propellant gas can be any liquefiable gas known in the art for use in aerosol containers. Examples of suitable propellants include trichlorofluoromethane, trichlorotrifluoroethane, monochlorodifluorormethane, difluoroethane, propane, butane, isobutane, used singly or in combination. In such product forms, the composition according to the invention may typically comprise 5-45%, and the propellant 55-95% by weight of the total composition.

Particularly preferred product forms of compositions according to the invention are creams, lotions, and sticks.

Depending on the product form, a number of other ingredients may be present in and form the balance of the topical compositions according to the invention. These may include:
- volatile and non volatile silicones, such as dimethyl cyclosiloxanes or polydimethyl siloxane, e.g., DOW CORNING fluids DC244, DC245, DC344, DC345, Q2 1465 and the 200 fluids;
- deoperfumes and deodorant compounds which can act as antimicrobial agents;
- minor amounts (for example less than about 10% by weight of the composition) of hydrophobic oils, such as liquid paraffin oils, isopropyl palmitate, and other emollients;
- thickening and gelling agents, such as clays, for example, Bentone 38, silicas, for example, Aerosil 200, organic waxes, such as castor wax, beeswax, or paraffin wax, silicone waxes, stearyl alcohol, fatty alcohols having 14-24 carbon atoms, fatty acids having 16-36 carbon atoms, propylene carbonate, and polyethylene;
- perfumes;
- preservatives and antioxidants;
- skin benefit agents, such as allantoin;
- humectants, such as polyols, for example glycerol;
- other cosmetic adjuncts conventionally employed in stick, roll on, propellant driven aerosol, pump spray and lotion products.

Particularly preferred optional components, in particular when the product form is a cream or lotion, include volatile silicones, which can preferably be present at a level of 40-60% by weight, propylene carbonate, which is preferably present at a level of 0.1-1% by weight, and silicas which are preferably present at a level of 2-8% by weight.

Preferably, the composition is essentially anhydrous; that is it contains no more than about 1% by weight of water.

### Examples

The invention will now be further described by way of example only.

### Example 1

The following compositions were prepared and packaged by conventional techniques. For the cream compositions, these are conveniently prepared by mixing together under high shear the liquid components of the composition, and then gradually adding under continued shear the powder elements of the composition. Finally, the silica in the composition is added under continued shear.

| | Composition (% w/w) | |
|---|---|---|
| Component | 1 | 2 |
| AAZG Powder (1) | 24.0 | 24.0 |
| Suprafino Talc | 5.5 | 10.0 |
| Volatile Silicone (2) | 8.0 | 8.0 |
| Non volatile silicone (3) | 14.0 | - |
| Silica (Aerosil 200) (4) | 4.0 | 2.0 |
| Polydecene (5) | 43.5 | 55.0 |
| Fragrance | 1.0 | 1.0 |

| | | |
|---|---|---|
| (1) Q5-7167, Activated aluminium zirconium glycinate, ex Summit | | |
| (2) Q2/1465, ex Dow Corning | | |
| (3) DC 200/350, ex Dow Corning | | |
| (4) ex Degussa | | |
| (5) Silkflo 364NF, viscosity 17 cSt@ 40°C, flash point >204°C, ex Albermarle Corporation. | | |

The above cream formulations were found to have remarkably good properties in terms of their ability to mask whiteness, the sensory feel on application and antiperspirant efficacy.

### Example 2

The following formulations provide suitable compositions according to the invention.

### Lotion

(Suitable for application from a roll on container)

| Component | % w/w |
|---|---|
| AAZG powder (6) | 25.0 |
| Polydecene (7) | 63.7 |
| Clay (8) | 3.0 |
| Polyethylene powder | 6.0 |
| Propylene Carbonate | 1.0 |
| Silica (9) | 0.3 |
| Fragrance | 1.0 |

| | |
|---|---|
| (6) Q5-7167, activated aluminium zirconium glycinate, ex Summit | |
| (7) Silkflo 364NF, ex Albermarle Corp | |
| (8) Bentone 38, ex Rheox | |
| (9) Aerosil 200, ex Degussa | |

### Stick

(Suitable for application from a barrel).

| Component | % w/w |
|---|---|
| AAZG Powder (6) | 24.0 |
| Polydecene (7) | 53.0 |
| Stearyl alcohol | 14.0 |
| Talc | 3.0 |
| Hydrogenated castorwax | 4.0 |
| Fragrance | 1.0 |
| PEG-8-Distearate | 1.0 |

### Lotion

| Component | % w/w |
|---|---|
| AAZG Powder (6) | 15.0 |
| Decamethylcyclopentasiloxane | 25.0 |
| Polydecene (7) | 54.5 |
| Clay (8) | 2.0 |
| Ethanol | 3.0 |
| Fragrance | 0.5 |

### Propellant driven aerosol base

| Component | % w/w |
|---|---|
| AACH (10) | 8.0 |
| Clay | 4.0 |
| PPG-14 Butyl Ether | 6.0 |
| Fragrance | 4.0 |
| Polydecene (7) | 78.0 |

| | |
|---|---|
| (10) Activated aluminium chlorohydrate, ex Guilini. | |

This aerosol base can be combined in a 1:3 ratio with hydrocarbon propellant to provide the product.

### Stick

| Component | % w/w |
|---|---|
| AAZG Powder (6) | 20.0 |
| Stearyl alcohol | 23.0 |
| Hydrogenated castor oil | 4.0 |
| Glyceryl monostearate | 1.0 |
| PPG-14 Butyl Ether | 2.0 |
| Magnesium aluminium silicate | 1.0 |
| Perfume | 1.0 |
| Cyclomethicone Pentamer blend | 17.0 |
| Polybutene (11) | 31.0 |

| | |
|---|---|
| (11) Panalene L14E, ex Amoco. | |

### Cream

| Component | % w/w |
|---|---|
| AAZG Powder (6) | 18.0 |
| Stearyl alcohol | 5.5 |
| Polydecene (7) | 45.0 |
| PEG-8-Distearate | 1.0 |
| Talc | 6.0 |
| Dimethicone | 14.0 |
| Cyclomethicone | 10.5 |

### Cream

| Component | % w/w |
|---|---|
| AAZG Powder (6) | 20.0 |
| Polyethylene Powder | 15.0 |
| Silica (9) | 4.0 |
| Propylene Carbonate | 0.5 |
| Dimethicone | 10.0 |
| Cyclomethicone | 8.0 |
| Polyisobutylene (12) | 42.50 |

| | |
|---|---|
| (12) Permethyl, ex Presperse | |

## Claims

1. An antiperspirant or deodorant composition for topical application to the human skin, comprising an antiperspirant or deodorant active material and a cosmetically suitable vehicle, the cosmetically suitable vehicle comprising 20-90% by weight of the total composition of a poly olefin emollient.

2. An antiperspirant or deodorant composition according to claim 1, wherein the poly olefin hydrocarbon is a poly alpha olefin.

3. An antiperspirant or deodorant composition acccording to claim 1 or claim 2, wherein the poly olefin hydrocarbon is liquid at room temperature.

4. An antiperspirant or deodorant composition according to any of the preceding claims, wherein the poly olefin hydrocarbon has a flash point of 140°C or higher.

5. An antiperspirant or deodorant composition according to any of the preceding claims, wherein the poly olefin hydrocarbon vehicle comprises 30-60% by weight of the composition.

6. An antiperspirant or deodorant composition according to any of the preceding claims, wherein the poly olefin is a polydecene.

7. An antiperspirant or deodorant composition according to any of the preceding claims, wherein the composition is in the form of a lotion, cream or stick.

8. An antiperspirant or deodorant composition according to any of the preceding claims in the form of an antiperspirant composition.
